(19) Europäisches Patentamt
European Patent Office
Office européen des brevets

(11) Veröffentlichungsnummer : **0 031 114**
**B1**

(12) **EUROPÄISCHE PATENTSCHRIFT**

(45) Veröffentlichungstag der Patentschrift : **21.03.84**

(51) Int. Cl.³ : **C 07 D295/02**, C 07 D295/06, C 07 D295/08, C 07 D211/14, A 01 N 43/34

(21) Anmeldenummer : **80107896.5**

(22) Anmeldetag : **13.12.80**

# (54) Phenylpropylammoniumsalze, Verfahren zu ihrer Herstellung und diese Verbindungen enthaltende Mittel.

(30) Priorität : **24.12.79 DE 2952382**

(43) Veröffentlichungstag der Anmeldung : **01.07.81 Patentblatt 81/26**

(45) Bekanntmachung des Hinweises auf die Patenterteilung : **21.03.84 Patentblatt 84/12**

(84) Benannte Vertragsstaaten : **AT BE CH DE FR GB IT LI NL SE**

(56) Entgegenhaltungen :
**EP-A- 0 002 604**
**EP-A- 0 007 479**
**DE-A- 2 752 096**
**R. Wegler, Chemie der Pflanzenschutz- und Schädlingsbekämpfungsmittel, Band 4, 1977, Springer-Verlag, Berlin, Seite 25**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Buschmann, Ernst, Dr.**
**Georg-Ludwig-Krebs-Strasse 10**
**D-6700 Ludwigshafen (DE)**
Erfinder : **Zeeh, Bernd, Dr.**
**Thorwaldsenstrasse 5**
**D-6700 Ludwigshafen (DE)**
Erfinder : **Jung, Johann, Dr.**
**Hardenburgstrasse 19**
**D-6703 Limburgerhof (DE)**
Erfinder : **Sauter, Hubert, Dr.**
**Neckarpromenade**
**D-6800 Mannheim (DE)**

EP 0 031 114 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Phenylpropylammoniumsalze, Verfahren zu ihrer Herstellung und diese Verbindungen enthaltende Mittel

Die vorliegende Erfindung betrifft quartäre Phenylpropylammoniumsalze, Verfahren zu ihrer Herstellung und Mittel, die diese Verbindungen enthalten und zur Regulierung des Pflanzenwachstums geeignet sind.

Es ist bekannt, daß 2-Chlorethyltrimethylammoniumchlorid, (J. Biol. Chem. *235,* 475 (1960)) pflanzenwachstums-regulierende Eigenschaften bei Getreide und anderen Kulturpflanzen aufweist (US-Patent 3 156 554). Weitere Ammoniumsalze mit wachstumsregulatorischer Wirkung sind bekannt u. a. aus DE-OS 20 17 497, 21 14 512 und 24 59 129 und aus R. WEGLER, Chemie der Pflanzenschutz- und Schädlingsbekämpfungsmittel, Band 4, 1977, Springer-Verlag, Berlin, Seite 25.

Bei der Anwendung der bekannten Mittel zur Regulierung des Pflanzenwachstums ist die Wirkung vor allem bei niedrigen Aufwandmengen und Konzentrationen oft nicht ausreichend.

Es wurden nun die neuen Phenylpropylammoniumsalze der Formel I gefunden,

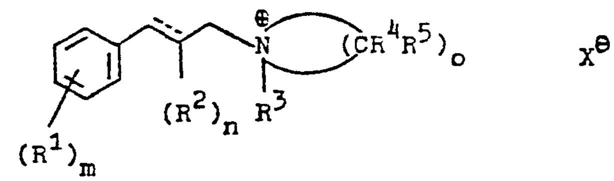

(I)

in der

$R^1$ einen Alkyl-, Cycloalkyl-, Alkoxyrest, Acyl mit jeweils bis zu 6 C-Atomen oder Halogen,

$R^2$ einen Alkyl-, Alkenyl- oder Alkoxyrest mit jeweils bis zu 6 C-Atomen,

$R^3$ einen $C_1$ bis $C_6$-Alkyl-, $C_3$ bis $C_6$-Alkenyl- oder Alkinylrest,

$R^4$ und $R^5$ Wasserstoff oder einen $C_1$ bis $C_3$-Alkylrest

m die Zahlen 0 bis 3

n die Zahlen 1 bis 2

o die Zahlen 4 bis 6 bedeuten und

$X^-$ für das Anion einer nicht-phytotoxischen Säure steht, wobei die gestrichelte Bindung für n = 1 hydriert sein kann und für n = 2 hydriert ist. Der Begriff Propyl umfaßt somit auch das einfach ungesättigte Propyl, nämlich Propenyl. Diese neuen Verbindungen als Wirkstoff enthaltende Mittel haben starke pflanzenwachstumsregulierende Eigenschaften.

$R^1$ bedeutet beispielsweise : 2-Methyl, 3-Methyl, 4-Methyl, 4-tert.Butyl, 3-Methyl-4-Chlor, 2-Methyl-4-chlor, 4-Cyclopentyl, 4-Cyclohexyl, 4-Methoxy, 4-Acetyl, 4-Propionyl, 2-Chlor, 4-Chlor, 2,4-$Cl_2$, 2,3,4-$Cl_3$, 2,4,5-$Cl_3$, 4-Brom, 2-Fluor.

$R^2$ bedeutet beispielsweise : Methyl, Ethyl, n-Propyl, iso-Propyl, n-Butyl, iso-Butyl, n-Pentyl, Propen-1-yl, Buten-1-yl, Methoxy, Ethoxy.

$R^3$ bedeutet beispielsweise Methyl, Ethyl, Propyl, n-Butyl, Iso-Butyl, Allyl, Crotyl, Propinyl, Butinyl.

$R^4$ und $R^5$ bedeuten beispielsweise Wasserstoff, Methyl, Ethyl, n-Propyl, Isopropyl.

Unter Berücksichtigung der Werte für o gleich 4 bis 6 ergibt somit der Rest

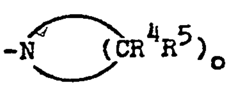

in den einfachsten Fällen, d. h. wenn $R^4$ und $R^5$ gleich Wasserstoff sind, Pyrrolidin, Piperidin und Hexamethylenimin. Diese sind wegen ihrer guten Zugänglichkeit bevorzugt. Wenn diese Heterocyclen substituiert sind ($R^4$ und/oder $R^5$ gleich $C_1$- bis $C_3$-Alkyl), sind solche bevorzugt, in denen 1 bis 3 der Methylenreste substituiert sind und jeweils nur einen Substituenten tragen. Wenn die Methylenreste zweifach substituiert sind, sind Methyl und Ethyl bevorzugt.

Die obere Grenze der Kohlenstoffzahl der Reste $R^1$ und $R^2$ ist nicht auf $C_5$ beschränkt. Bevorzugt sind Reste mit 1 bis 4 Kohlenstoffatomen, doch kommen biespielsweise auch Reste mit 6 Kohlenstoffatomen in Betracht.

Unter nicht-phytotoxischen Anionen sind solche zu verstehen, die in den Aufwandmengen des Wirkstoffes dessen Brauchbarkeit nicht beeinträchtigen und somit phytotoxisch unbedenklich sind. Phytotoxisch unbedenkliche Anionen, für die $X^{\ominus}$ steht, sind insbesondere Jod, Brom, Chlor, Sulfat, Tosylat, Mesylat, Dodecylsulfonat, Dodecylphenylsulfonat, 4-Bromphenylsulfonat, da vorzugsweise diese sich bei der Herstellung der Verbindungen ergeben. Sie können dann auf übliche Weise ausgetauscht werden, z. B. durch die Anionen Nitrat, Phosphat, Acetat, Benzoat. Die Anionen können demnach ein oder mehrwertig, anorganisch oder organisch sein.

Die erfindungsgemäßen Verbindungen können als Wirkstoffe in Wachstumsregulatoren verwendet werden. Die erfindungsgemäßen Mittel zeigen überraschend eine wesentlich stärkere pflanzenwachstumsregulierende Wirkung als das bekannte 2-Chlorethyl-trimethylammoniumchlorid, das ein anerkannt guter Stoff gleicher Wirkungsart ist.

Ausgangsverbindungen für die Herstellung der neuen Phenyl-propylammoniumsalze der Formel I sind die teilweise aus DE-OS 27 52 096 bekannten Phenylpropylamine der Formel II,

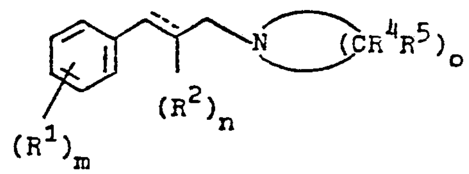

(II)

in der $R^1$, $R^2$, $R^4$, $R^5$, m, n und o die oben genannten Bedeutungen haben. Diese tertiären Amine lassen sich in an sich üblicher Weise mit Quaternierungsmitteln zu den Endverbindungen umsetzen. Als Quaternierungsmittel kommen außer den Alkyl-, Alkenyl-, Alkinyl-, $R^3$-Halogeniden z. B. in Betracht : Dimethylsulfat. Diethylsulfat und Sulfonsäureester der Formel $R—SO_3R^3$, wobei R $C_1$ bis $C_{20}$-Alkyl und durch Halogen oder Alkyl substituiertes Phenyl bedeuten kann.

Alternative Ausgangsverbindungen sind die Phenylpropylhalogenide der Formel III,

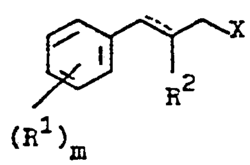

(III)

in der $R^1$, $R^2$ und m die oben genannten Bedeutungen haben und in der X Cl, Br oder J bedeutet, die ebenfalls teilweise aus DE-OS 27 52 096 bekannt sind.

Das folgende Schema beschreibt, wie die Verbindungen II und III mit allgemein bekannten Reaktionen hergestellt werden können. Die Herstellung der Aldehyde IV ist beschrieben von B. Zeeh und E. Buschmann in Liebigs Ann. Chem. 1979, S. 1585.

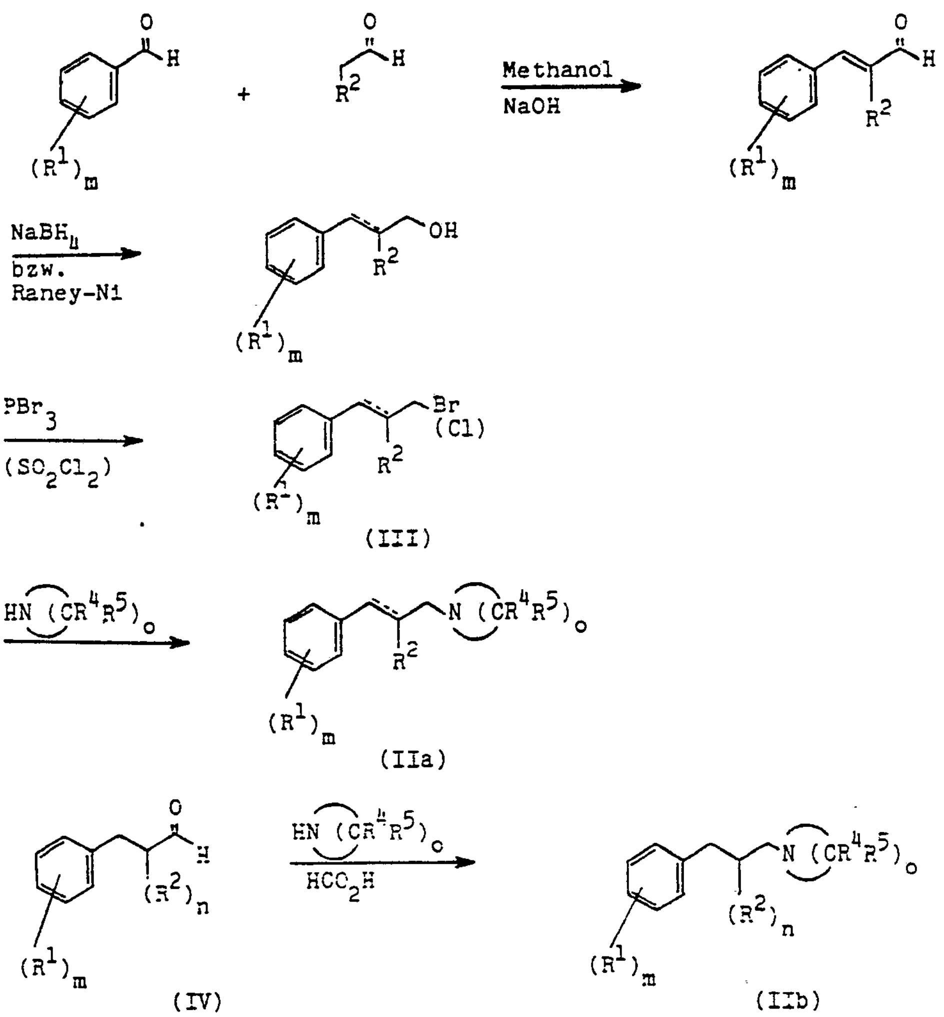

(III)
(IIa)
(IV)
(IIb)

Aus dem Schema ergibt sich, daß man die neuen Phenylpropylammoniumsalze erhält, indem man ein Phenylpropylamin der Formel II mit einem Alkylierungsmittel der Formel $R^3X$, wobei $R^3$ und X die oben genannten Bedeutungen haben, umsetzt. Alternativ erhält man die erfindungsgemäßen Wirkstoffe indem man ein Phenylpropylhalogenid der Formel III mit tertiären Aminen der Formel V

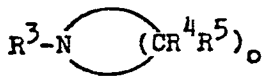

in der $R^3$, $R^4$ und $R^5$ und o die obengenannten Bedeutungen haben, umsetzt.

Die Umsetzung der Phenylpropylamine II mit $R^3X$ und die Umsetzung der Phenylpropylhalogenide III mit den tertiären Aminen V erfolgt beispielsweise bei einer Temperatur von 10 bis 150 °C in Anwesenheit oder Abwesenheit eines Lösungsmittels, z. B. Ethanol, Methanol, $CHCl_3$, $CH_2Cl_2$, Acetonitril, Dimethylformamid oder Essigester bei Normaldruck oder erhöhtem Druck.

Die folgenden Beispiele erläutern die Herstellung der erfindungsgemäßen Verbindungen.

N-Crotonyl-N-[3-(2,4-dichlorphenyl)-2-methyl-2-prop-1-en-1-yl-propyl]-pyrrolidiniumbromid. (Beispiel Nr. 20).

Eine Lösung von 31,2 g N-[3-(2,4-Dichlorphenyl)-2-methyl-2-prop-1-en-1-yl-propyl]-pyrrolidin und 27 g Crotylbromid in 250 ml Essigester wird 5 h zum Rückfluß erwärmt. Man rührt 15 h bei Raumtemperatur, saugt das kristallin angefallene Produkt ab, wäscht mit Ether und trocknet im Vakuum. Man erhält 15 g N-Crotonyl-N-[3-(2,4-dichlor-phenyl)-2-methyl-2-prop-1-en-1-yl-propyl-] pyrrolidinium-bromid, Schmelzpunkt 128 °C.

N-(3-(2,4-Dichlorphenyl)-2-methyl-propyl)-N-methyl-hexamethylenimmoniumbromid (Beispiel Nr. 29).

In 300 ml Acetonitril werden 19 g Methylbromid eingegast. Nach Zugabe von 30 g N-[3-(2,4-Dichlorphenyl)-2-methyl-propyl]-hexamethylenimin und 15 h Rühren bei Raumtemperatur wird eingeengt. Der Rückstand kristallisiert nach Verreiben mit Essigester. Das Rohprodukt wird abgesaugt, mit Essigester gewaschen und im Vakuum getrocknet. Man erhält 18 g N-[3-(2,4-Dichlorphenyl)-2-methyl-propyl]-N-methyl-hexamethylenimmoniumbromid. Schmelzpunkt 178 °C.

N-Allyl-N-[2-methyl-3-(o-methyl-phenyl)-propyl]-pyrrolidiniumbromid (Beispiel Nr. 24).

Eine Lösung von 51 g N-[2-Methyl-3-(o-methyl-phenyl)-propyl]-pyrrolidin und 58 g Allylbromid in 200 ml Essigester wird 5 h zum Rückfluß erwärmt. Das Produkt fällt als Harz aus. Nach Abdekantieren des Essigesters wird das Rohprodukt mehrfach mit Essigester verrieben. Das überstehende Lösungsmittel wird abdekantiert. Zuletzt wird mit n-Pentan verrieben und abdekantiert. Das zurückbleibende Öl wird am Rotationsverdampfer von Lösungsmittelresten befreit. Es verbleiben 60 g N-Allyl-N-[2-methyl-3-(o-methyl-phenyl)-propyl]-pyrrolidiniumbromid als braunes Harz.

Auf entsprechende Weise sind die aus folgender Tabelle ersichtlichen Verbindungen erhältlich :

Tabelle 1

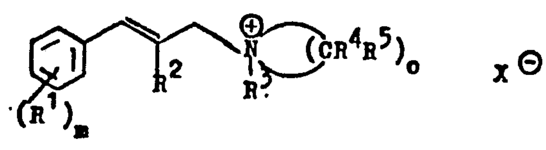

| Beispiel Nr. | $(R^1)_m$ | $R^2$ | $R^3$ | $(CR^4R^5)_o$ | X | Schmp. |
|---|---|---|---|---|---|---|
| 1 | 4-Cl | $CH_3$ | Allyl | $(CH_2)_4$ | Br | |
| 2 | 2,4-Cl | $CH_3$ | Allyl | $(CH_2)_4$ | Br | 110° |
| 3 | 2,4-Cl | $C_2H_5$ | Allyl | $(CH_2)_4$ | Br | |
| 4 | 2,4-Cl | $n-C_4H_9$ | Allyl | $(CH_2)_4$ | Br | |
| 5 | 2,4-Cl | $n-C_5H_{11}$ | Allyl | $(CH_2)_4$ | Br | 109° |
| 6 | H | $CH_3$ | Allyl | $(CH_2)_4$ | Br | |
| 7 | 2,4-Cl | Isopropyl | Allyl | $(CH_2)_4$ | Br | 75° |
| 8 | 2,3,4-Cl | $n-C_4H_9$ | Allyl | $(CH_2)_4$ | Br | |
| 9 | 2,4-Cl | $OCH_3$ | Allyl | $(CH_2)_4$ | Br | |
| 10 | 2,4-Cl | $n-C_5H_{11}$ | Allyl | $(CH_2)_5$ | Br | 148° |

(Fortsetzung)

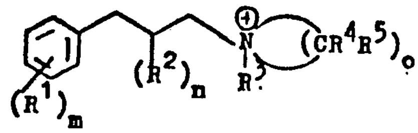

$X^{\ominus}$

| Beispiel Nr. | $(R^1)_m$ | $(R^2)_n$ | $R^3$ | $(CR^4R^5)_o$ | X | Schmp. |
|---|---|---|---|---|---|---|
| 11 | 2-F | $CH_3$ | Allyl | $(CH_2)_4$ | Br | Harz |
| 12 | 4-Acetyl | $CH_3$ | Allyl | $(CH_2)_4$ | Br | Harz |
| 13 | 2,4-Cl | $CH_3$ | Allyl | $(CH_2)_4$ | Br | Harz |
| 14 | 2,4-Cl | $CH_3$ | Allyl | $(CH_2)_4$ | Cl | |
| 15 | 4-$OCH_3$ | $CH_3$ | Allyl | $(CH_2)_4$ | Br | Harz |
| 16 | 2,4-Cl | iso-Propyl | Allyl | $(CH_2)_4$ | Br | 123° |
| 17 | 2,4-Cl | n-Propyl | Allyl | $(CH_2)_4$ | Br | Harz |
| 18 | 2,4-Cl | Methyl Propen-1-yl | Crotyl | $(CH_2)_4$ | Br | 128° |
| 19 | 3-$CH_3$, 4-Cl | $CH_3$ | Allyl | $(CH_2)_4$ | Br | Harz |
| 20 | 4-Cl | $CH_3$ Propen-1-yl | Allyl | $(CH_2)_4$ | Br | 147° |
| 21 | 2,4-Cl | $(CH_3)_2$ | Allyl | $(CH_2)_4$ | Br | 136-8° |
| 22 | 2-$CH_3$ | $CH_3$ | Allyl | $(CH_2)_4$ | Br | Harz |
| 23 | 3-$CH_3$ | $CH_3$ | Allyl | $(CH_2)_4$ | Br | |
| 24 | 2-Cl | $CH_3$ | Allyl | $(CH_2)_4$ | Br | 105° |
| 25 | 2,4-Cl | Methyl Propen-1-yl | Allyl | $(CH_2)_4$ | Br | 172-4° |
| 26 | 2,4-Cl | $CH_3$ | Ethyl | $(CH_2)_6$ | Br | 176-8° |
| 27 | 2,4-Cl | $CH_3$ | Methyl | $(CH_2)_6$ | Br | 178° |
| 28 | 2,4-Cl | $CH_3$ | Allyl | $(CH_2)_6$ | Br | 177-180° |
| 29 | 4-Cl | $CH_3$ | Allyl | $(CH_2)_6$ | Br | 76-77° |
| 30 | 4-Cl | $CH_3$ | Allyl | $(CH_2)_5$ | Br | 55-58° |
| 31 | 4-t-Butyl | $CH_3$ | Allyl | $(CH_2)_4$ | Br | 125° |
| 32 | 2,4-Cl | $CH_3$ | $CH_3$ | $(CH_2)_4$ | Br | Harz |
| 33 | H | $CH_3$ | Allyl | $(CH_2)_4$ | Br | Harz |
| 34 | 4-Cl | $CH_3$ | Allyl | $(CH_2)_4$ | Br | Harz |
| 35 | 2,3,4-Cl | $CH_3$ | Allyl | $(CH_2)_4$ | Br | |
| 36 | 2,3,4-Cl | n-$C_4H_9$ | Allyl | $(CH_2)_4$ | Br | Harz |
| 37 | 2,3,4-Cl | Isopropyl | Allyl | $(CH_2)_4$ | Br | |
| 38 | 2,4-Cl | $CH_3$ | Crotyl | $(CH_2)_4$ | Br | Harz |
| 39 | 2,4-Cl | n-Propyl | Allyl | $(CH_2)_4$ | J | |
| 40 | 2,4-Cl | $OCH_3$ | Allyl | $(CH_2)_4$ | Br | Harz |

(Fortsetzung)

| Beispiel Nr. | $(R^1)_n$ | $(R^2)_n$ | $R^3$ | $(CR^4R^5)_o$ | X | Schmp. |
|---|---|---|---|---|---|---|
| 41 | 2,4-Cl | $CH_3$ | Allyl | $(CH_2)_3$-<br>$-CHCH_3CH_2$ | Br | |
| 42 | 2,4-Cl | $n-C_3H_7$ | Propinyl | $(CH_2)_4$ | Br | |
| 43 | 4-Br | $CH_3$ | Allyl | $(CH_2)_4$ | Br | Harz |
| 44 | $4-CH_3$ | $CH_3$ | Allyl | $(CH_2)_4$ | Br | Harz |
| 45 | 2,4-Cl | $n-C_3H_7$ | Allyl | $(CH_2)_5$ | Br | Harz |

Für die Wirkungsweise von Pflanzenwachstumsregulatoren gilt allgemein, daß sie mehrere verschiedenartige Wirkungen auf Pflanzen ausüben können. Die Wirkungen der Stoffe hängen im wesentlichen ab von dem Zeitpunkt der Anwendung, bezogen auf das Entwicklungsstadium des Samens oder der Pflanze, sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwachstumregulierende Stoffe können z. B. zur Hemmung des vegetativen Pflanzenwachstums eingesetzt werden. Eine derartige Wuchshemmung ist u. a. bei Gräsern von wirtschaftlichem Interesse, denn durch eine Dämpfung des Graswachstums kann z. B. die Häufigkeit des Grasmähens in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, wozu unter anderem Reis gehört. Denn durch eine Halmverkürzung wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung, so daß ein Mehrertrag bezogen auf die Bodenfläche erzielt werden kann.

Ein weiterer Mechanismus der Ertragssteigerung mit Pflanzenwachstumsregulatoren beruht darauf, daß die Nährstoffe in stärkerem Maße der Blüten- und Fruchtbildung zugute kommen, während das vegetative Wachstum eingeschränkt wird.

Mit Pflanzenwachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, so daß z. B. mehr oder größere Früchte zur Ausbildung kommen.

Ertragssteigerungen können in manchen Fällen auch durch einen Eingriff in den pflanzlichen Stoffwechsel erreicht werden, ohne daß sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluß von Wachstumsregulatoren kann es zur Ausbildung parthenokarper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Ernte, z. B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, — z. B. bei Obst —, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß

eine mechanische Beerntung der Pflanzen ermöglicht beziehungsweise eine manuelle Beerntung erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Hilfe von Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z. B. um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz bei Pflanzen induziert werden.

Die unterschiedelichen Kulturen, in denen Wachstumsregulatoren angewendet werden können, die verschiedenen Wirkungsziele, Boden- und Witterungsverhältnisse machen es verständlich, daß es nicht möglich ist, die erforderlichen Aufwandmengen für jeden einzelnen Fall anzugeben. Dem Fachmann bereitet es aber keine Schwierigkeiten, die am meisten geeigneten Aufwandmengen festzustellen, da die brauchbare höchste Menge im allgemeinen bei 5 kg Wirkstoff je Hektar liegt. Bei Soja beispielsweise können ungefähr 0,25 kg/ha bis ungefähr 2,5 kg/ha im Nachauflaufverfahren ausgebracht werden.

Die Mittel werden beispielsweise in Form von direkt versprühbaren Lösungen, Pulvern, Suspensionen, auch als hochprozentige wäßrige, ölige oder sonstige Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt wie Kerosin oder Dieselöl, ferner Kohlenteeröle, sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser in Betracht.

Da es sich bei den Wirkstoffen dieser Erfindung um Salze handelt sind wäßrige Anwendungsformen bevorzugt. Sie können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Wirkstoffe als solche oder in einem Öl oder Lösungsmittel gelöst, mittels Netz-, Haft-, Dispergier- oder Emulgiermittel in Wasser homogenisiert werden. Es können aber auch aus Wirkstoff Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht : Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol, Octylphenol-, Nonylphenol, Alkylphenolpolyglykolether, Tributylphenylpolyglykolether, Alkylarylpolyetheralkohole, Isotridecylalkohol, Fettalkohol-ethylenoxid-Kondensate, ethoxyliertes Rizinusöl, Polyoxyäthylenalkylether, ethoxyliertes Polyoxypropylen, Laurylalkoholpolylglykoletheracetal, Sorbitester, Lignin, Sulfitablaugen und Methylcellulose.

Pulver, Streu- und Stäubemittel können durch Mischen oder gemeinsames Vermahlen der Wirkstoffe mit einem festen Trägerstoff hergestellt werden.

Granulate z. B. Umhüllungs-, Imprägnierungs- und Homogengranulate, können durch Bindung der Wirkstoffe an festen Trägerstoffe hergestellt werden. Feste Trägerstoffe sind Mineralerden wie Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel,

wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die erfindungsgemäßen Mittel können zusätzlich weitere Wirkstoffe enthalten, z. B. Herbizide und/oder Fungizide.

Die Ergebnisse von biologischen Tests mit den erfindungsgemäßen Mitteln weisen eine sehr bemerkenswerte wachstumsregulierende Wirkung aus, wie die in den Tabellen wiedergegebenen Daten beispielhaft zeigen. Dazu wurden Testpflanzen auf ausreichend mit Nährstoffen versorgtem Kultursubstrat in Kunststoffgefäßen von ungefähr 12,5 cm Durchmesser angezogen. Im Nachauflaufverfahren wurden die zu prüfenden neuen Verbindungen in wäßriger Aufbereitung auf die Pflanzen versprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Vergleichssubstanz :

$$CH_3-\overset{CH_3}{\underset{CH_3}{N^+}}-CH_2-CH_2-Cl \qquad Cl$$

Tabelle 2

Sojabohnen

Nachauflaufverfahren

| Beispiel Nr. | Konz. mg WS/Gef. | Wuchshöhe % |
|---|---|---|
| unbeh. | - | 100 |
| Vergleichssubstanz | 1,5 | 96,5 |
| | 6,0 | 88,8 |
| 17 | 1,5 | 50,0 |
| | 6,0 | 39,3 |
| 13 | 1,5 | 61,8 |
| | 6,0 | 45,9 |
| 32 | 1,5 | 67,7 |
| | 6,0 | 51,8 |
| 38 | 1,5 | 65,7 |
| | 6,0 | 51,8 |
| 26 | 1,5 | 58,4 |
| | 6,0 | 52,0 |
| 40 | 1,5 | 48,7 |
| | 6,0 | 42,2 |
| 33 | 1,5 | 60,1 |
| | 6,0 | 55,1 |

WS = Wirkstoff
Gef. = Gefäß

Tabelle 3

Sonnenblumen

Nachauflaufverfahren

| Beispiel Nr. | Konz. mg WS/Gef. | Wuchshöhe % |
|---|---|---|
| unbeh. | - | 100 |
| Vergleichssubstanz | 1,5 | 89,5 |
| | 6,0 | 81,7 |
| 17 | 1,5 | 70,3 |
| | 6,0 | 58,6 |
| 13 | 1,5 | 69,0 |
| | 6,0 | 61,3 |
| 32 | 1,5 | 70,0 |
| | 6,0 | 64,2 |
| 43 | 1,5 | 75,1 |
| | 6,0 | 61,4 |
| 40 | 1,5 | 75,1 |
| | 6,0 | 61,4 |

Tabelle 4

Sonnenblumen

Nachauflaufverfahren

| | Nr.d. chem. Beisp. | Konz. mg WS/Gef. | Wuchshöhen % |
|---|---|---|---|
| unbehandelt | - | - | 100 |
| Vergleichssubstanz | - | 1,5 | 93,8 |
| | | 6 | 85,9 |
| | 7 | 1,5 | 89,3 |
| | | 6 | 71,4 |
| | 19 | 1,5 | 62,3 |
| | | 6 | 56,8 |
| | 22 | 1,5 | 80,1 |
| | | 6 | 70,3 |
| | 44 | 1,5 | 63,4 |
| | | 6 | 56,0 |
| | 24 | 1,5 | 70,9 |
| | | 6 | 57,8 |

Tabelle 5

Sojabohnen

Nachauflaufverfahren

| | Nr.d.chem. Beisp. | Konz. mg WS/Gef. | Wuchshöhen % |
|---|---|---|---|
| unbehandelt | - | - | 100 |
| Vergleichssubstanz | - | 0,5 | 97,1 |
| | | 1,5 | 97,1 |
| | 19 | 0,5 | 80,2 |
| | | 1,5 | 73,8 |
| | 22 | 0,5 | 78,1 |
| | | 1,5 | 69,6 |

**Ansprüche** (für die Vertragsstaaten : BE, CH, DE, FR, GB, IT, LI, NL, SE)

1. Phenylpropylammoniumsalze der Formel I

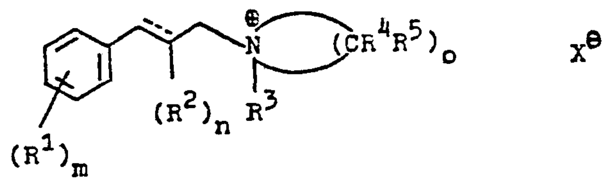

(I)

in der

$R^1$ einen Alkyl-, Cycloalkyl-, Alkoxyrest, Acyl mit jeweils bis zu 6 C-Atomen oder Halogen,
$R^2$ einen Alkyl-, Alkenyl- oder Alkoxyrest mit jeweils bis zu 6 C-Atomen,
$R^3$ einen $C_1$ bis $C_6$-Alkyl-, $C_3$ bis $C_6$-Alkenyl- oder Alkinylrest,
$R^4$ und $R^5$ Wasserstoff oder einen $C_1$ bis $C_3$-Alkylrest,
m die Zahlen 0 bis 3,
n die Zahlen 1 bis 2,
o die Zahlen 4 bis 6 bedeuten und
$X^-$ für das Anion einer nicht-phytotoxischen Säure steht, wobei die gestrichelte Bindung für n = 1 hydriert sein kann und für n = 2 hydriert ist.

2. Phenylpropylammoniumsalze gemäß Anspruch 1 der Formel Ia

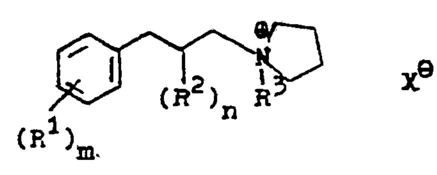

(Ia)

in der $R^1$, $R^2$, $R^3$, m, n und X die obengenannten Bedeutungen haben, wobei die gestrichelte Bindung für n = 1 hydriert sein kann und für n = 2 hydriert ist.

3. Phenylpropylammoniumsalze gemäß Anspruch 1 der Formel Ib

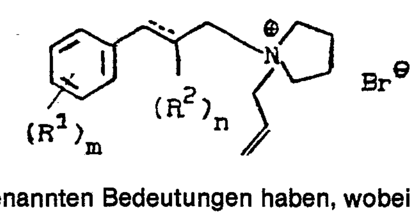

(Ib)

in der $R^1$, $R^2$, m und n die obengenannten Bedeutungen haben, wobei die gestrichelte Bindung für n = 1 hydriert sein kann und für n = 2 hydriert ist.

4. Phenylpropylammoniumsalze gemäß Anspruch 1 der Formel Ic

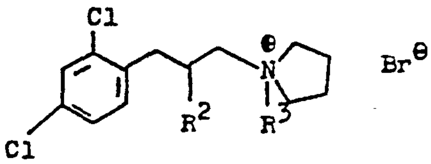

(Ic)

in der $R^2$ und $R^3$ die obengenannten Bedeutungen haben.

5. Phenylpropylammoniumsalz gemäß Anspruch 1, ausgewählt aus der Gruppe, bestehend aus

N-Allyl-N-[3-(2-Fluorphenyl)-2-methylpropyl]-pyrrolidiniumbromid,
N-Allyl-N-[3-(2,4-dichlorphenyl)-2-methylpropyl]-pyrrolidiniumbromid,
N-[3-(2,4-Dichlorphenyl)-2-methyl-propyl]-n-methyl-pyrrolidiniumbromid,
N-Allyl-N-(3-phenyl-2-methyl-propyl)-pyrrolidiniumbromid und
N-Allyl-N-[3-(4-chlorphenyl)-2-methyl-propyl]-pyrrolidiniumbromid.

6. Phenylpropylammoniumsalz gemäß Anspruch 1, ausgewählt aus der Gruppe, bestehend aus

N-Allyl-N-[3-(2,4-dichlorphenyl)-2-isopropyl-propyl]-pyrrolidiniumbromid und
N-Allyl-n-[3-(2,4-dichlorphenyl)-2-propyl-propyl]-pyrrolidiniumbromid.

7. Verfahren zur Herstellung von Verbindungen gemäß Anspruch 1, dadurch gekennzeichnet, daß man aus einer Verbindung der Formel II

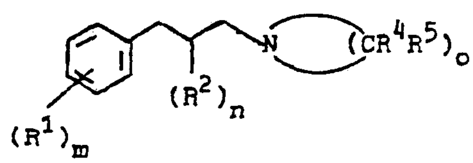

(II)

das Ammoniumsalz der Formel I herstellt oder eine Verbindung der Formel III

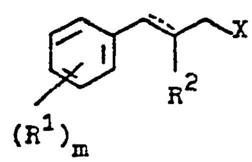

(III)

mit einem tertiären Amin der Formel V

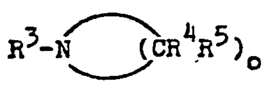

(V)

zum Ammoniumsalz der Formel I umsetzt und gewünschtenfalls das Anion austauscht, wobei die Substituenten die in Anspruch 1 angegebenen Bedeutungen haben.

8. Mittel zur Regulierung des Pflanzenwachstums, enthaltend ein Phenylpropylammoniumsalz gemäß Anspruch 1.

**Ansprüche** (für den Vertragsstaat AT)

1. Mittel zur Regulierung des Pflanzenwachstums, enthaltend ein Phenylpropylammoniumsalze der Formel I

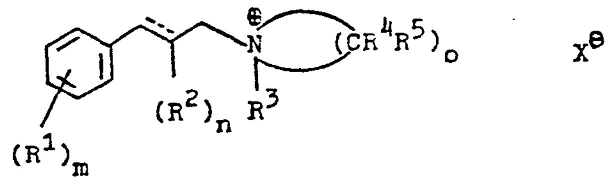

(I)

in der

$R^1$ einen Alkyl-, Cycloalkyl-, Alkoxyrest, Acyl mit jeweils bis zu 6 C-Atomen oder Halogen,
$R^2$ einen Alkyl-, Alkenyl- oder Alkoxyrest mit jeweils bis zu 6 C-Atomen,
$R^3$ einen $C_1$ bis $C_6$-Alkyl-, $C_3$ bis $C_6$-Alkenyl- oder Alkinylrest,

$R^4$ und $R^5$ Wasserstoff oder einen $C_1$ bis $C_3$-Alkylrest,
m die Zahlen 0 bis 3,
n die Zahlen 1 bis 2,
o die Zahlen 4 bis 6 bedeuten und
$X^-$ für das Anion einer nicht-phytotoxischen Säure steht, wobei die gestrichelte Bindung für n = 1 hydriert sein kann und für n = 2 hydriert ist.

2. Mittel zur Regulierung des Pflanzenwachstums, enthaltend ein Phenylpropylammoniumsalze gemäß Anspruch 1 der Formel Ia

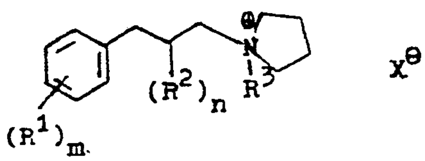

(Ia)

in der $R^1$, $R^2$, $R^3$, m, n und X die obengenannten Bedeutungen haben, wobei die gestrichelte Bindung für n = 1 hydriert sein kann und für n = 2 hydriert ist.

3. Mittel zur Regulierung des Pflanzenwachstums, enthaltend ein Phenylpropylammoniumsalze gemäß Anspruch 1 der Formel Ib

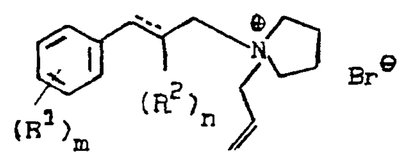

(Ib)

in der $R^1$, $R^2$, m und n die obengenannten Bedeutungen haben, wobei die gestrichelte Bindung für n = 1 hydriert sein kann und für n = 2 hydriert ist.

4. Mittel zur Regulierung des Pflanzenwachstums, enthaltend ein Phenylpropylammoniumsalze gemäß Anspruch 1 der Formel Ic

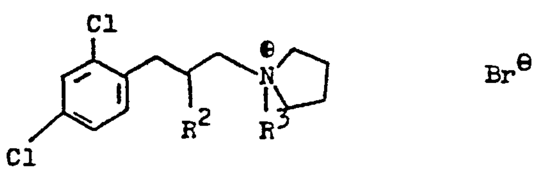

(Ic)

in der $R^2$ und $R^3$ die obengenannten Bedeutungen haben.

5. Mittel zur Regulierung des Pflanzenwachstums gemäß Anspruch 1, enthaltend ein Phenylpropylammoniumsalz, ausgewählt aus der Gruppe, bestehend aus

N-Allyl-N-[3-(2-Fluorphenyl)-2-methylpropyl]-pyrrolidiniumbromid,
N-Allyl-N-[3-(2,4-dichlorphenyl)-2-methylpropyl]-pyrrolidiniumbromid,
N-[3-(2,4-Dichlorphenyl)-2-methyl-propyl]-n-methyl-pyrrolidiniumbromid,
N-Allyl-N-(3-phenyl-2-methyl-propyl)-pyrrolidinium-bromid und
N-Allyl-N-[3-(4-chlorphenyl)-2-methyl-propyl]-pyrrolidiniumbromid.

6. Mittel zur Regulierung des Pflanzenwachstums gemäß Anspruch 1, enthaltend ein Phenylpropylammoniumsalz, ausgewählt aus der Gruppe, bestehend aus

N-Allyl-N-[3-(2,4-dichlorphenyl)-2-isopropyl-propyl]-pyrrolidiniumbromid und
N-Allyl-N-[3-(2,4-dichlorphenyl)-2-propyl-propyl]-pyrrolidiniumbromid.

7. Verfahren zur Herstellung von Verbindungen gemäß Anspruch 1, dadurch gekennzeichnet, daß man aus einer Verbindung der Formel II

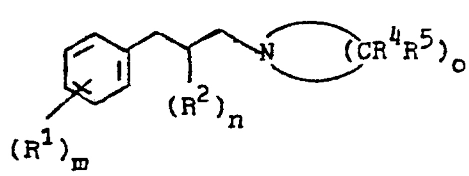

(II)

das Ammoniumsalz der Formel I herstellt oder eine Verbindung der Formel III

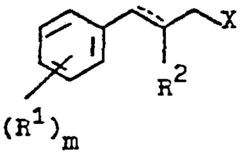

(III)

mit einem tertiären Amin der Formel V

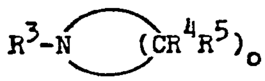

(V)

zum Ammoniumsalz der Formel I umsetzt und gewünschtenfalls das Anion austauscht, wobei die Substituenten die in Anspruch 1 angegebenen Bedeutungen haben.

8. Mittel zur Regulierung des Pflanzenwachstums, enthaltend ein Phenylpropylammoniumsalz gemäß Anspruch 1.

**Claims** (for the Contracting States : BE, CH, DE, FR, GB, IT, LI, NL, SE)

1. A phenylpropylammonium salt of the formula I

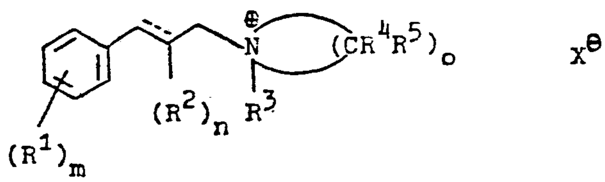

(I)

where

$R^1$ denotes alkyl, cycloalkyl, alkoxy or acyl, each having up to 6 carbon atoms, or halogen,
$R^2$ denotes alkyl, alkenyl or alkoxy, each having up to 6 carbon atoms,
$R^3$ denotes $c_1$-$C_6$-alkyl, $C_3$-$C_6$-alkenyl or alkynyl,
$R^4$ and $R^5$ denote hydrogen or $C_1$-$C_3$-alkyl,
m denotes one of the integers 0, 1, 2 and 3,
n denotes one of the integers 1 and 2,
o denotes one of the integers 4, 5 and 6,
$X^-$ denotes the anion of a non-phytotoxic acid, and the dashed bond may be hydrogenated when n is 1, and is hydrogenated when n is 2.

2. A phenylpropylammonium salt as claimed in claim 1, of the formula Ia

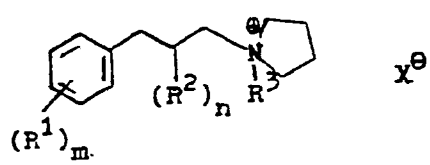

(Ia)

where $R^1$, $R^2$, $R^3$, m, n and X have the above meanings, and the dashed bond may be hydrogenated when n is 1, and is hydrogenated when n is 2.

3. A phenylpropylammonium salt as claimed in claim 1, of the formula Ib

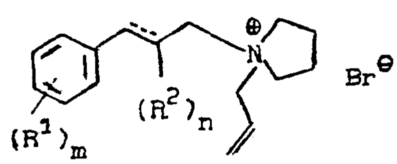

(Ib)

where $R^1$, $R^2$, m and n have the above meanings, and the dashed bond may be hydrogenated when n is 1, and is hydrogenated when n is 2.

4. A phenylpropylammonium salt as claimed in claim 1, of the formula Ic

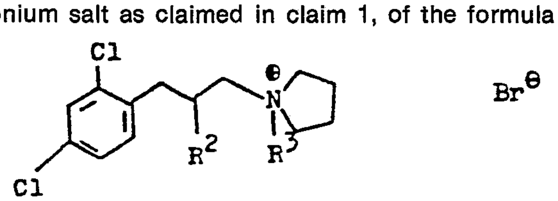

(Ic)

where $R^2$ and $R^3$ have the above meanings.

5. A phenylpropylammonium salt as claimed in claim 1, selected from the group consisting of

N-allyl-N-[3-(2-fluorophenyl)-2-methylpropyl]-pyrrolidinium bromide,
N-allyl-N-[3-(2,4-dichlorophenyl)-2-methylpropyl]-pyrrolidinium bromide,
N-[3-(2,4-dichlorophenyl)-2-methyl-propyl]-n-methyl-pyrrolidinium bromide,
N-allyl-N-(3-phenyl-2-methyl-propyl)-pyrrolidinium bromide and
N-allyl-N-[3-(4-chlorophenyl)-2-methyl-propyl]-pyrrolidinium bromide.

6. A phenylpropylammonium salt as claimed in claim 1, selected from the group consisting of

N-allyl-N-[3-(2,4-dichlorophenyl)-2-isopropyl-propyl]-pyrrolidinium bromide and
N-allyl-N-[3-(2,4-dichlorophenyl)-2-propyl-propyl]-pyrrolidinium bromide.

7. A process for the manufacture of a compound as claimed in claim 1, wherein the ammonium salt of the formula I is prepared from a compound of the formula II

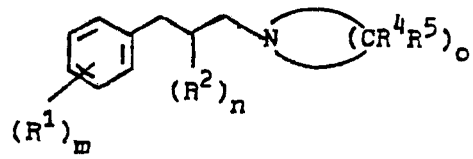

(II)

or a compound of the formula III

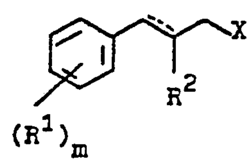

(III)

is reacted with a tertiary amine of the formula V

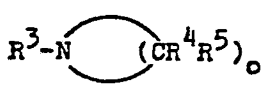

(V)

to give the ammonium salt of the formula I, and the anion is if desired exchanged, the substituents having the meanings given in claim 1.

8. An agent for regulating plant growth, containing a phenylpropylammonium salt as claimed in claim 1.

**Claims** (for the Contracting State AT)

1. An agent for regulating plant growth, containing a phenylpropylammonium salt of the formula I

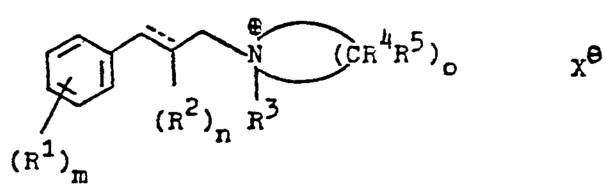

(I)

where

$R^1$ denotes alkyl, cycloalkyl, alkoxy or acyl, each having up to 6 carbon atoms, or halogen,
$R^2$ denotes alkyl, alkenyl or alkoxy, each having up to 6 carbon atoms,
$R^3$ denotes $C_1$-$C_6$-alkyl, $C_3$-$C_6$-alkenyl or alkynyl,
$R^4$ and $R^5$ denote hydrogen or $C_1$-$C_3$-alkyl,
m denotes one of the integers 0, 1, 2 and 3,
n denotes one of the integers 1 and 2,
o denotes one of the integers 4, 5 and 6,
$X^-$ denotes the anion of a non-phytotoxic acid, and the dashed bond may be hydrogenated when n is 1, and is hydrogenated when n is 2.

2. An agent for regulating plant growth, containing a phenylpropylammonium salt as defined in claim 1, of the formula Ia

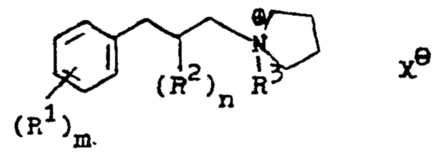

(Ia)

where $R^1$, $R^2$, $R^3$, m, n and X have the above meanings, and the dashed bond may be hydrogenated when n is 1, and is hydrogenated when n is 2.

3. An agent for regulating plant growth, containing a phenylpropylammonium salt as defined in claim 1, of the formula Ib

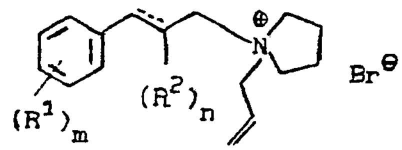

(Ib)

where $R^1$, $R^2$, m and n have the above meanings, and the dashed bond may be hydrogenated when n is 1, and is hydrogenated when n is 2.

4. An agent for regulating plant growth, containing a phenylpropylammonium salt as defined in claim 1, of the formula Ic

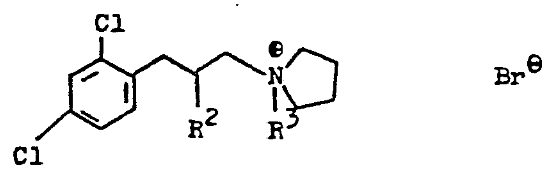

(Ic)

where $R^2$ and $R^3$ have the above meanings.

5. An agent for regulating plant growth as claimed in claim 1, containing a phenylpropylammonium salt selected from the group consisting of

N-allyl-N-[3-(2-fluorophenyl)-2-methylpropyl]-pyrrolidinium bromide,
N-allyl-N-[3-(2,4-dichlorophenyl)-2-methylpropyl]-pyrrolidinium bromide,
N-[3-(2,4-dichlorophenyl)-2-methyl-propyl]-n-methyl-pyrrolidinium bromide,
N-allyl-N-(3-phenyl-2-methyl-propyl)-pyrrolidinium bromide and
N-allyl-N-[3-(4-chlorophenyl)-2-methyl-propyl]-pyrrolidinium bromide.

6. An agent for regulating plant growth as claimed in claim 1, containing a phenylpropylammonium salt selected from the group consisting of

N-allyl-N-[2,4-dichloro-phenyl)-2-isopropyl-propyl]-pyrrolidinium bromide and
N-allyl-N-[3-(2,4-dichlorophenyl)-2-propyl-propyl]-pyrrolidinium bromide.

7. A process for the manufacture of a compound as defined in claim 1, wherein the ammonium salt of the formula I is prepared from a compound of the formula II

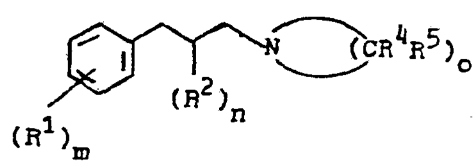

(II),

or a compound of the formula III

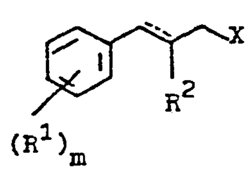

(III)

is reacted with a tertiary amine of the formula V

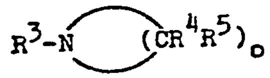

(V)

to give the ammonium salt of the formula I, and the anion is if desired exchanged, the substituents having the meanings given in claim 1.

8. An agent for regulating plant growth, containing a phenylpropylammonium salt as defined in claim 1.

**Revendications** (pour les Etats Contractants : BE, CH, DE, FR, GB, IT, LI, NL, SE)

1. Sels de phénylpropyl-ammonium de la formule I

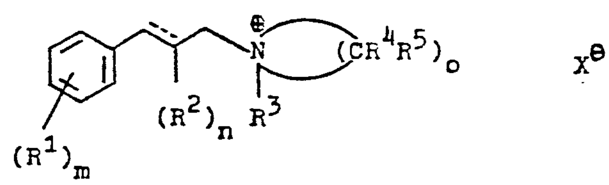

(I)

dans laquelle

$R^1$ désigne un groupe alkyle, cycloalkyle, alcoxy ou acyle avec jusqu'à 6 atomes de carbone ou un atome d'halogène,

$R^2$ désigne un groupe alkyle, alcényle ou alcoxy avec jusqu'à 6 atomes de carbone,

$R^3$ représente un radical alkyle en $C_1$ à $C_6$, alcényle en $C_3$ à $C_6$ ou alcynyle,

$R^4$ et $R^5$ sont soit des atomes d'hydrogène, soit des radicaux alkyle en $C_1$ à $C_3$,

m est un nombre de 0 à 3,

n est un nombre valant 1 ou 2,

o est un nombre de 4 à 6 et

$X^-$ désigne un anion d'un acide non phyto-toxique, la liaison en trait interrompu pouvant être hydrogénée, lorsque n = 1, et étant hydrogénée pour n = 2.

2. Sels de phénylpropyl-ammonium selon la revendication 1, de la formule Ia

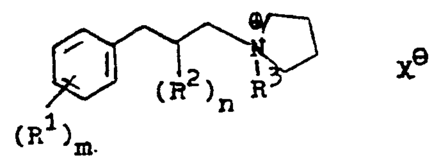

(Ia)

dans laquelle $R^1$, $R^2$, $R^3$, m, n et X possèdent les significations définies, la liaison en trait interrompu pouvant être hydrogénée, lorsque n = 1, et étant hydrogénée pour n = 2.

3. Sels de phénylpropyl-ammonium selon la revendication 1, de la formule Ib

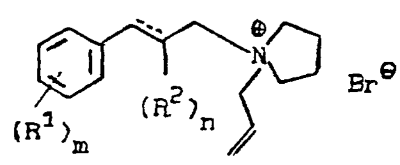

(Ib)

dans laquelle $R^1$, $R^2$, m et n possèdent les significations définies, la liaison en trait interrompu pouvant être hydrogénée, lorsque n = 1 et étant hydrogénée pour n = 2.

4. Sels de phénylpropyl-ammonium selon la revendication 1, de la formule Ic

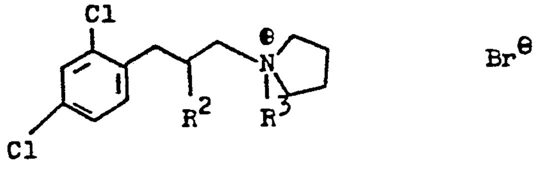

(Ic)

dans laquelle $R^2$ et $R^3$ possèdent les significations définies.

5. Sel de phénylpropyl-ammonium selon la revendication 1, choisi parmi

le bromure de N-allyl-N-[3-(2-fluorophényl)-2-méthyl-propyl]-pyrrolidinium,
le bromure de N-allyl-N-[3-(2,4-dichlorophényl)-2-méthyl-propyl]-pyrrolidinium,
le bromure de N-[3-(2,4-dichlorophényl)-2-méthyl-propyl]-n-méthyl-pyrrolidinium,
le bromure de N-allyl-N-(3-phényl-2-méthyl-propyl)-pyrrolidinium et
le bromure de N-allyl-N-[3-(4-chlorophényl)-2-méthyl-propyl]-pyrrolidinium.

6. Sel de phénylpropyl-ammonium selon la revendication 1, choisi parmi

le bromure de N-allyl-n-[3-(2,4-dichlorophényl)-2-iso-propyl-propyl]-pyrrolidinium et
le bromure de N-allyl-n-[3-(2,4-dichlorophényl)-2-propyl-propyl]-pyrrolidinium.

7. Procédé de préparation de composés selon la revendication 1, caractérisé en ce que l'on transforme un composé de la formule II

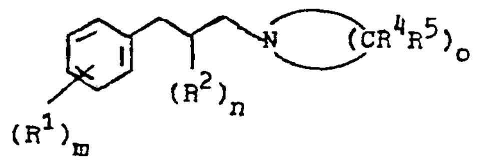

(II)

en sel d'ammonium de la formule I ou que l'on fait réagir un composé de la formule III

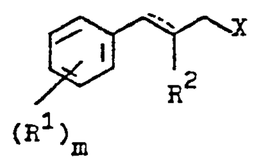

(III)

avec une amine tertiaire de la formule V

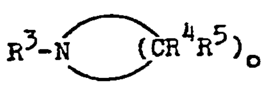

(V)

pour obtenir un sel d'ammonium de la formule I, dont l'anion peut éventuellement être remplacé par un autre, les substituants dans les formules ci-dessus possédant les significations définies dans la revendication 1.

8. Composition pour la régulation de la croissance des plantes, contenant un sel de phénylpropyl-ammonium selon la revendication 1.

**Revendications** (pour l'Etat Contractant AT)

1. Composition pour la régulation de la croissance des plantes, contenant un sel de diphénylpropyl-ammonium de la formule I

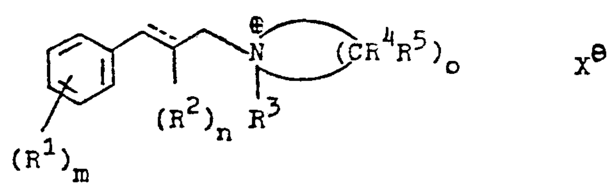

(I)

dans laquelle

$R^1$ désigne un groupe alkyle, cycloalkyle, alcoxy ou acyle avec jusqu'à 6 atomes de carbone ou un atome d'halogène,

$R^2$ désigne un groupe alkyle, alcényle ou alcoxy avec jusqu'à 6 atomes de carbone,

$R^3$ représente un radical alkyle en $C_1$ à $C_6$, alcényle en $C_3$ à $C_6$ ou alcynyle,

$R^4$ et $R^5$ sont soit des atomes d'hydrogène, soit des radicaux alkyle en $C_1$ à $C_3$,

m est un nombre de 0 à 3,

n est un nombre valant 1 ou 2,

o est un nombre de 4 à 6 et

$X^-$ désigne un anion d'un acide non phyto-toxique, la liaison en trait interrompu pouvant être hydrogénée, lorsque n = 1, et étant hydrogénée pour n = 2.

2. Composition pour la régulation de la croissance des plantes, contenant un sel de phénylpropylammonium selon la revendication 1, de la formule Ia

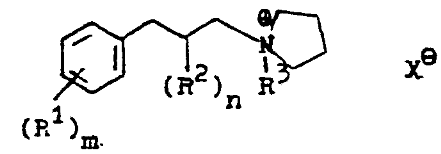

(Ia)

dans laquelle $R^1$, $R^2$, $R^3$, m, n et X possèdent les significations définies, la liaison en trait interrompu pouvant être hydrogénée, lorsque n = 1, et étant hydrogénée pour n = 2.

3. Composition pour la régulation de la croissance des plantes, contenant un sel de phénylpropylammonium selon la revendication 1, de la formule Ib

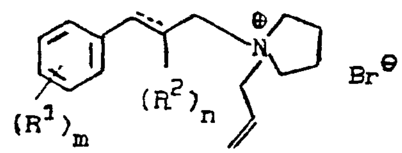

(Ib)

dans laquelle $R^1$, $R^2$, m et n possèdent les significations définies, la liaison en trait interrompu pouvant être hydrogénée, lorsque n = 1 et étant hydrogénée pour n = 2.

4. Composition pour la régulation de la croissance des plantes, contenant un sel de phénylpropylammonium selon la revendication 1, de la formule Ic

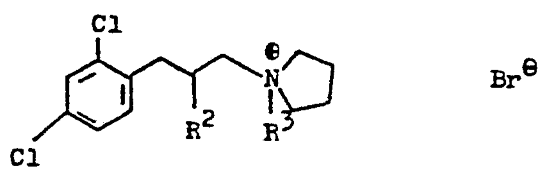

(Ic)

dans laquelle $R^2$ et $R^3$ possèdent les significations définies.

5. Composition pour la régulation de la croissance des plantes, contenant un sel de phénylpropylammonium selon la revendication 1, choisi parmi

le bromure de N-allyl-N-[3-(2-fluorophényl)-2-méthyl-propyl]-pyrrolidinium,
le bromure de N-allyl-N-[3-(2,4-dichlorophényl)-2-méthyl-propyl]-pyrrolidinium,
le bromure de N-[3-(2,4-dichlorophényl)-2-méthyl-propyl]-n-méthyl-pyrrolidinium,
le bromure de N-allyl-N-(3-phényl-2-méthyl-propyl)-pyrrolidinium et
le bromure de N-allyl-N-[3-(4-chlorophényl)-2-méthyl-propyl]-pyrrolidinium.

6. Composition pour la régulation de la croissance des plantes selon la revendication 1, contenant un sel de phénylpropyl-ammonium choisi parmi

le bromure de N-allyl-N-[3-(2,4-dichlorophényl)-2-iso-propyl-propyl]-pyrrolidinium et
le bromure de N-allyl-N-[3-(2,4-dichlorophényl)-2-propyl-propyl]-pyrrolidinium.

7. Procédé de préparation de composés selon la revendication 1, caractérisé en ce que l'on transforme un composé de la formule II

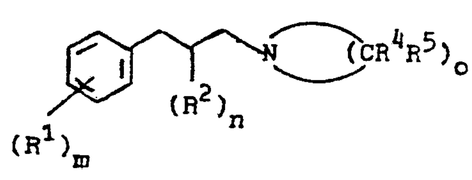

(II)

en sel d'ammonium de la formule I ou que l'on fait réagir un composé de la formule III

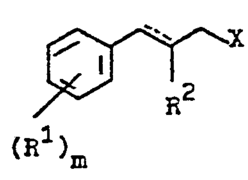

(III)

avec une amine tertiaire de la formule V

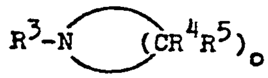

(V)

pour obtenir un sel d'ammonium de la formule I, dont l'anion peut éventuellement être remplacé par un autre, les substituants dans les formules ci-dessus possédant les significations définies dans la revendication 1.

8. Composition pour la régulation de la croissance des plantes, contenant un sel de phénylpropylammonium selon la revendication 1.